# EUROPÄISCHE PATENTANMELDUNG

(11) **EP 0 815 863 A1**
(43) Veröffentlichungstag der Anmeldung: **07.01.1998**
(21) Anmeldenummer: 97110901.2
(22) Anmeldetag: 02.07.1997
(51) Int. Cl.: A61K 31/70

(54) **Kombination von S-Adenosylmethionin und Azidothymidin zur Hemmung der HIV-Replikation**

(30) Priorität: 05.07.1996 DE 19628514
(71) Anmelder: Grundmann, Ewald, Dr., 45136 Essen (DE)
(72) Erfinder: Grundmann, Ewald, Dr., 45136 Essen (DE)
(74) Vertreter: Wolgast, Rudolf, Dipl.-Chem. Dr.

(57) **Zusammenfassung**

Die HIV-Replikation in einer HIV-infizierten Lymphocytenkultur wird durch die Menge des erzeugten viralen Hüllproteins p24 in Gegenwart der Hemmstoffe S-Adenosylmethionin und Azidothymidin (3'-Azido-2',3'-disdesoxythymidin) bestimmt. Dabei wird gefunden, daß in Anwesenheit beider Hemmstoffe die Hemmwirkung größer ist als die Hemmwirkung jedes der einzelnen Hemmstoffe.

## Beschreibung

Die Erfindung betrifft Mittel zur Hemmung der HIV-Replikation, sowie die Verwendung einer Kombination von S-Adenosylmethionin und Azidothymidin zur Hemmung der HIV-Replikation.

Aus dem europäischen Patent 0 516 660 des gleichen Anmelders ist bekannt, daß S-Adenosylmethionin in seiner stabilisierten Form als Tosylat-bis-sulfat zur Herstellung eines Arzneimittels gegen virale Infektionen durch Retroviren, insbesondere AIDS-Viren verwendet werden kann, da diese Substanz eine hohe Wirksamkeit gegen solche Viren hat. So ergaben in vitro-Versuche an mit AIDS-Viren infizierten Lymphocytenkulturen humanen Ursprungs, daß die Bildung der für die Viren charakteristischen Reversen Transcriptase in Gegenwart von S-Adenosylmethionin in einer Halbwertskonzentration im Bereich von 100 bis 200 µg/ml gehemmt wird.

Aus einer Veröffentlichung (Jon H. Condra et al., Nature 1995, Bd. 374, S. 569-571) ist bekannt, daß Inhibitoren für die HIV-1-Protease die HIV-Replikation hemmen, wobei einzelne Proteaseinhibitoren oder Kombinationen verschiedener Proteaseinhibitoren eingesetzt werden können. Jedoch entwickeln sich rasch resistente HIV-1-Varianten, wobei besonders nachteilhaft ist, daß dabei eine Kreuzresistenz entsteht, indem ein einzelner Proteaseinhibitor auch zur Ausbildung von Varianten führt, die auch gegen andere einzelne Proteaseinhibitoren resistent sind. Die Aufgabe der Erfindung besteht darin, ein Mittel zur Hemmung der HIV-Replikation zu schaffen, das stärker wirksam ist als S-Adenosylmethionin allein.

Überraschenderweise wurde nun gefunden, daß ein Mittel, das S-Adenosylmethionin und Azidothymidin enthält, eine im Vergleich zu den Einzelkomponenten gesteigerte Hemmwirkung für die HIV-Replikation hat.

Unter S-Adenosylmethionin wird hier die stabilisierte Substanz in Form des Tosylat-bis-sulfats verstanden; "Azidothymidin" ist eine Kurzbezeichnung für das unter der Abkürzung AZT bekannte AIDS-Mittel, das chemisch 3'-Azido-2',3'-didesoxythymidin ist.

Die erfindungsgemäße Kombination zeichnet sich dadurch aus, daß die Komponenten (im Unterschied zu den vorerwähnten Proteaseinhibitoren) unterschiedliche Wirkungsweisen bzw. Wirkungsorte haben, wodurch die Entstehung von Kreuzresistenzen vermieden wird. Selbst bei der Entstehung einer Resistenz gegen eine der Komponenten wird daher die Wirksamkeit der anderen Komponente nicht oder kaum beeinflußt.

Das erfindungsgemäße Mittel kann in einer physiologisch verträglichen Menge, insbesondere in einem verträglichen Träger verteilt sein, der zusätzlich übliche Zusatz- und Hilfsstoffe enthalten kann.

Die Erfindung umfaßt auch die Verwendung einer Kombination von S-Adenosylmethionin und Azidothymidin zur Herstellung eines Arzneimittels für die Hemmung der HIV-Replikation.

In der Praxis hat sich gezeigt, daß Azidothymidin bei der Behandlung von AIDS-Infektionen eine Reihe von nachteiligen Nebenwirkungen hat. Ein besonderer Vorteil des erfindungsgemäßen Mittels besteht darin, daß bei Verwendung beider Substanzen die Dosis von Azidothymidin und damit auch dessen nachteilige Nebenwirkungen herabgesetzt werden können, ohne daß seine Wirksamkeit dadurch verringert wird.

Die Erfindung wird nachstehend an Hand eines Beispiels erläutert, das sich auf in vitro-Untersuchungen bezieht.

Die Untersuchungen wurden an menschlichen peripheren Lymphocytenkulturen durchgeführt, die mit HIV-1-Viren des Prototyps HIV IIIB infiziert wurden. Dabei wurden die einzelnen Komponenten als solche und die Komponenten in verschiedene Mengenverhältnissen geprüft. Die Prüfung erfolgte durch die Bestimmung des in den Kulturen gebildeten Virus-Kernproteins p24. Im einzelnen wurde dabei wie folgt verfahren:
Menschliche periphere Lymphocyten wurden durch Dichtegradienten-Zentrifugierung aus Blutproben von gesunden Probanden isoliert. Die sogenannte buffy coat fraction wurde im Verhältnis 1:1 mit einer sterilen, phosphatgepufferten Kochsalzlösung (nachfolgend als "PBS" abgekürzt; die Lösung enthält 50 mM Natriumphosphat in 0,9%iger Kochsalzlösung von pH 7,4) verdünnt und auf Ficoll geschichtet. Nach 30 min Zentrifugieren bei 400 g wird der Überstand verworfen und die Zwischenphase, welche die Lymphocyten enthält, isoliert. Die so erhaltenen Lymphocyten werden zweimal mit PBS gewaschen und schließlich in ein Zellkulturmedium aufgenommen.

Die Lebensfähigkeit der Zellen wurde durch Färbung nach dem Trypanblau-Ausschlußverfahren geprüft; dadurch wurde die Gesamtkonzentration der Zellen und der Prozentsatz der lebensfähigen Zellen in der Suspension bestimmt. Die Zellkonzentration in der Suspension wurde dann auf 1x10⁷/ml eingestellt. Diese Zellsuspension wurde dann auf ein Zellkulturgefäß (Wuchsfläche: 75 cm²) übertragen, und die Zellen wurden durch Zugabe von Phytohämagglutinin (Endkonzentration von 5 µg/ml) polyklonal aktiviert. Die Zellen wurden dann 48 Stdn. lang in befeuchteter Atmosphäre bei 37°C und mit einem CO₂-Gehalt von 5% kultiviert. Nach dieser Inkubation wurden die Zellen durch Zentrifugieren geerntet und wieder in Zellkulturmedium aufgenommen.

Die Lymphocyten wurden alsdann in einem Sicherheitslaboratorium der Stufe P3 mit einem Standardpräparat des Isolats HIV IIIB eines Protyps von HIV-1 infiziert. Dazu wurden die Zellen durch Zentrifugieren sedimentiert, der Überstand wurde verworfen und die Zellen wurden wiederum im HIV-Impfmittel suspendiert. Das Impfmittel ist auf Konzentrationen von 100 ng/ml des viralen Kernproteins p24 und 3200 pg/ml HIV-Ribonukleinsäure eingestellt.

3x10⁸ Lymphocyten wurden in 1 ml des Impfmittels suspendiert und 60 min bei 37°C inkubiert. Anschließend wurden die Zellen zweimal mit 50 ml des Kulturmediums gewaschen und erneut in einer Konzentration von 3x10⁶ Zellen pro ml in einem Kulturmedium suspendiert, das 10 Einheiten/ml menschlichen rekombinanten Interleukins-2 enthielt. 100 µl einer vorverdünnten Lösung der zu untersuchenden Verbindung wurden mit 100 µl dieser Zellsuspension versetzt. Anschließend wurde der gesamte Ansatz in befeuchteter Atmosphäre bei 37°C in Gegenwart von 5% CO₂ bebrütet.

Am vierten Tage nach der Infizierung wurde die virale Replikation in den Zellkulturen bestimmt. Endpunkt ist die Bestimmung von viralem Kernprotein p24 im Überstand jeder Zellkultur. Dazu wurden 150 µl des Überstandes abgenommen und in ein Gefäß übertragen, das pro Vertiefung (well) 50 µl von 0,08%igem Natriumdodecylsulfat (SDS) enthielt. Zur Bestimmung des viralen Kernproteins p24, das von den infizierten Zellen produziert wurde, diente das bekannte "Sandwich ELISA" Verfahren, vgl. Ch. Müller, R. Moritz,G. Prigge, D. Weitzel, R. Kunze, A. Kage und E. Köttgen, 1988, Fresenius Z.Anal.Chem. 330, S. 352-353.

Es wurden jeweils 3 Proben untersucht; Mittelwerte der jeweils dreifachen Bestimmung sind in der nachfolgenden Tabelle zusammengefaßt.

**Tabelle**

| ng p24/ml in HIV-infizierten Lymphocyten in Gegenwart von S-Adenosylmethionin und Azidothymidin | | | | | | | |
|---|---|---|---|---|---|---|---|
| SAM* | 0 | 0,1 | 0,2 | 1 | 5 | 10 | 100 AZT** |
| 0 | 33,0 | 28,3 | 32,3 | 30,5 | 15,7 | 13,2 | 3,5 |
| 10 | 33,9 | | 32,3 | 31,8 | 20,8 | | |
| 33 | 22,5 | | 22,1 | 18,5 | 6,9 | | |
| 100 | 13,4 | | 10,5 | 7,6 | 2,7 | | |
| 330 | 5,9 | | 4,1 | 1,8 | 0,9 | | |
| 1000 | 0,9 | | 0,2 | 0 | 0 | | |

| | | | | | | | |
|---|---|---|---|---|---|---|---|
| In der vorstehenden Tabelle bedeuten: SAM* = µg/ml S-Adenosylmethionin-Tosylat-bis-sulfat; und | | | | | | | |
| AZT** = ng/ml 3'-Azido-2',3'-didesoxythymidin | | | | | | | |

Aus der vorstehenden Tabelle ergibt sich folgendes: Die bei niedrigen wie bei hohen Konzentrationen von SAM und AZT gefundenen Werte für p24 variieren sehr stark, was wahrscheinlich auf Ungenauigkeit der Messung zurückzuführen ist. Bei mittleren Werten im Bereich von >10 µg/ml bis ≤330 µg/ml SAM und von >0,2 bis <100 ng/ml AZT unterstützen sich SAM und AZT gegenseitig, wie sich daraus ergibt, daß die Produktion von p24, also die HIV-Replikation in Gegenwart von SAM und AZT stärker gehemmt ist als in Gegenwart von nur SAM oder nur AZT. Die in Gegenwart beider Komponenten gefundene Hemmung ist jedoch stärker als sie sich aus der Summe der Hemmwirkungen der Komponenten ergeben würde:

So beträgt die Hemmung 33% in Gegenwart von 33 µg/ml SAM, aber in Abwesenheit von AZT; in Gegenwart von 1 ng/ml AZT, aber in Abwesenheit von SAM beträgt die Hemmung 10%; in Gegenwart von beiden ist die Hemmwirkung des SAM um 18% erhöht. In Gegenwart von 100 µg/ml SAM beträgt die Hemmung 59%, 0,2 ng/ml AZT bewirken selbst keine Hemmung, während in Gegenwart von beiden die Hemmwirkung des SAM um 22% erhöht ist; bei gleicher SAM-Konzentration und in Gegenwart von 1 ng/ml AZT, das 10% Hemmung bewirkt, ist die Hemmwirkung von SAM um 43% erhöht. In Gegenwart von 330 µg/ml SAM beträgt die Hemmung 82%, 0,2 ng/ml AZT bewirken selbst keine Hemmung, in Gegenwart von beiden ist die Hemmwirkung von SAM um 30% erhöht; bei gleicher SAM-Konzentration und in Gegenwart von 1 ng/ml AZT, das 10% Hemmung bewirkt, ist die Hemmwirkung von SAM um 70% erhöht.

Entsprechend beträgt die Hemmung 0% in Gegenwart von 0,2 ng/ml AZT und 59% in Gegenwart von 100 µg/ml SAM, in Gegenwart von beiden ist die Hemmwirkung stark erhöht, und gleiches gilt in Gegenwart 330 µg/ml SAM. In Gegenwart von 1 ng/ml AZT beträgt die Hemmung 10%, bei zusätzlicher Anwesenheit von 33 µg/ml SAM, das selbst eine Hemmung von 33% bewirkt, ist die Hemmwirkung von AZT um 58% erhöht; bei der gleichen AZT-Konzentration und zusätzlicher Anwesenheit von 100 µg/ml SAM, das selbst eine Hemmung von 59% bewirkt, ist die Hemmwirkung von AZT ist um 41% erhöht; bei der gleichen AZT-Konzentration und in Gegenwart von 330 µg/ml SAM, das selbst eine Hemmung von 95% bewirkt, ist die Hemmwirkung des AZT um 24% erhöht.

## Patentansprüche

1. Mittel zur Hemmung der HIV-Replikation, dadurch gekennzeichnet, daß das Mittel S-Adenosylmethionin und Azidothymidin enthält.

2. Mittel zur Hemmung der HIV-Replikation nach Anspruch 1, dadurch gekennzeichnet, daß das S-Adenosylmethionin und Azidothymidin in dem Mittel in physiologisch verträglichen Mengen enthalten ist.

3. Mittel nach Anspruch 1 oder 2, dadurch gekennzeichnet, daß das S-Adenosylmethionin und Azidothymidin in einem verträglichen Träger verteilt sind.

4. Mittel nach Anspruch 3, dadurch gekennzeichnet, daß in dem Träger weitere verträgliche, übliche Zusatz- und Hilfsstoffe enthalten sind.

5. Verwendung einer Kombination von S-Adenosylmethionin und Azidothymidin zur Herstellung eines Arzneimittels für die Hemmung der HIV-Replikation.
